(19) **Europäisches Patentamt**

**European Patent Office**

**Office européen des brevets**

(11) **EP 1 180 007 B1**

(12) **EUROPEAN PATENT SPECIFICATION**

(45) Date of publication and mention
of the grant of the patent:
**05.04.2006 Bulletin 2006/14**

(21) Application number: **00931233.1**

(22) Date of filing: **12.05.2000**

(51) Int Cl.:
*A61Q 5/00* (2006.01)     *A61K 8/64* (2006.01)

(86) International application number:
**PCT/EP2000/004420**

(87) International publication number:
**WO 2000/071086 (30.11.2000 Gazette 2000/48)**

(54) **USE OF THE COMBINATION OF AMINO ACIDS HAVING AN ALIPHATIC SIDE CHAIN AND HAVING A BASIC SIDE CHAIN IN TOPICAL HAIR TREATMENT**

VERWENDUNG EINER KOMBINATION VON AMINOSÄUREN MIT ALIPHATISCHER SEITENKETTE UND MIT BASISCHER SEITENKETTE IN TOPISCHEN HAARBEHANDLUNGSMITTELN

UTILISATION D'UNE COMBINAISON D'ACIDES AMINES A CHAINE LATERALE ALIPHATIQUE ET A CHAINE LATERALE BASIQUE DANS LE TRAITEMENT LOCAL DES CHEVEUX

(84) Designated Contracting States:
**AT BE CH CY DE DK ES FI FR GB GR IE IT LI LU MC NL PT SE**

(30) Priority: **24.05.1999 GB 9912085**

(43) Date of publication of application:
**20.02.2002 Bulletin 2002/08**

(73) Proprietors:
• **UNILEVER PLC**
**London EC4P 4BQ (GB)**
Designated Contracting States:
**CY GB IE**
• **UNILEVER N.V.**
**3013 AL Rotterdam (NL)**
Designated Contracting States:
**AT BE CH DE DK ES FI FR GR IT LI LU MC NL PT SE**

(72) Inventor: **TUCCORI, Sonia Regina Mangiavacchi Wirral,**
**Merseyside CH63 3JW (GB)**

(74) Representative: **Tansley, Sally Elizabeth et al**
**Unilever Patent Group**
**Colworth House**
**Sharnbrook**
**Bedford, MK44 1LQ (GB)**

(56) References cited:
EP-A- 0 681 826         EP-A- 0 747 035
WO-A-97/25972          DE-A- 4 022 288
DE-A- 19 617 569        DE-A- 19 808 395
GB-A- 1 408 036

• PATENT ABSTRACTS OF JAPAN vol. 1999, no. 12, 29 October 1999 (1999-10-29) & JP 11 180836 A (AJINOMOTO CO INC), 6 July 1999 (1999-07-06) & JAPANESE PATENT OFFICE, [Online] Retrieved from the Internet: <URL:http: //www4.jpdl.jpo-miti.go.jp/cgi-b in/tran_web_cgi-ejje> [retrieved on 2000-10-25]
• DATABASE WPI Section Ch, Week 199836 Derwent Publications Ltd., London, GB; Class A96, AN 1998-422272 XP002151790 & JP 10 175824 A (LION CORP), 30 June 1998 (1998-06-30)
• PATENT ABSTRACTS OF JAPAN vol. 1998, no. 08, 30 June 1998 (1998-06-30) & JP 10 059829 A (NOEVIR CO LTD), 3 March 1998 (1998-03-03)
• CODERCH, L. ET AL.: "Percutaneous penetration in vivo of amino acids" INTERNATIONAL JOURNAL OF PHARMACEUTICS, vol. 111, no. 1, 1994, page 7-14 XP000953258

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

FIELD OF THE INVENTION

This invention relates to the use in topical hair treatment compositions, such as shampoos and conditioners, of certain selected amino acids as ingredients for the repair and/or prevention of split ends in hair.

BACKGROUND OF THE INVENTION

Split ends are a phenomenon which is manifested in the hair shaft becoming porous and splitting at the ends. Split ends are caused, among other reasons, by severe mechanical stressing of the hair, for example by frequent brushing, backcombing or combing against a high combing resistance. High combing resistance in dry hair can be caused by damage to the hair surface, static charging or tackiness due to the residue of styling aids such as hair spray resins. The risk of split ends is also increased by weakening of the hair structure which can be caused by frequent use of harsh chemical treatments such as permanent waves, bleaching preparations or colorants.

Accordingly, there has been no shortage of attempts to treat hair damaged by split ends to halt the further progress of splitting and to restore the damaged hair to a healthy appearance. Various cosmetic preparations are known for repairing damaged hair, including hot oil treatments to replace the oils removed by shampoo, and many other hair conditioners that are designed to repair the damage caused by the harsh chemical treatments described above.

However, such cosmetic preparations are unsatisfactory in many ways. Many of the treatments contain chemical compounds that are not needed to repair the hair.

Such unneeded chemical compounds may cause additional damage to the hair or coat the hair in a manner that adversely impacts the appearance of the hair.

Other treatments target specific types of damage and may require combination with other cosmetic treatments to repair all of the damage to the hair. Interactions between the various cosmetic treatments may create additional difficulties. Persons having sensitive skin may experience adverse reactions to the chemicals present within these cosmetic treatments.

We have now found that certain selected amino acids are particularly effective for the repair and/or prevention of split ends in hair. Advantageously, these are safe and natural ingredients which are simple to formulate into topical hair treatment compositions such as shampoos and conditioners and furthermore do not build up on or coat the hair in the way described above with respect to prior art formulations intended for the treatment of split ends in hair.

Amino acids are known to be important for the nourishment of the human hair root and the growth of human hair.

GB 1 401 089 describes hair treatment compositions containing natural amino acids or protein hydrolysates. Particularly preferred are glycine, glutamic acid, aspartic acid, lysine, serine and alanine, for the reason that these amino acids are naturally found combined, in the form of polypeptides, in the substances collagen and keratin which occur as structural components of human skin and hair. A shampoo composition is described which incorporates protein hydrolysates resulting from the complete hydrolysis of collagen.

EP 0 186 025 discloses cosmetic preparations for care of skin and hair with a hydrolysate of almond protein. Almond protein is characterised by a high content of acidic and basic amino acids and contains 40-50 wt% glutamic acid, aspartic acid and arginine.

Numerous publications describe the use of an array of amino acids, often in "cocktail" form, in lotions or tonics for topical application to cure baldness and other skin, scalp and hair disorders. Examples of such disclosures are JP 59/007,111 which relates to a hair cosmetic with dandruff-preventing properties containing vitamin E acetate and methionine, CA 888,689 which describes hair and scalp preparations for normalisation of scalp secretions and promotion of hair growth which may contain, inter alia, cysteine and methionine in a base incorporating bergamot oil and nonionic emulsifier, and WO 92/00720 which describes a composition containing L-leucine, L-isoleucine and L-valine, said to stimulate growth and regeneration of hair and nails.

The prior art discussed above does not address the repair and/or prevention of split ends in hair by a combination of certain selected amino acids.

SUMMARY OF THE INVENTION

The present invention provides the use in a topical hair treatment composition of a combination of

(i) an amino acid having an aliphatic side chain; and
(ii) an amino acid having a basic side chain

for the repair and/or prevention of split ends in hair.

## DETAILED DESCRIPTION OF THE INVENTION

### Amino acid (i)

Amino acids (i) having an aliphatic side chain may be selected from glycine, alanine, valine, leucine and isoleucine, and mixtures thereof. Glycine has a hydrogen atom as its side chain, alanine has a methyl group, and larger hydrocarbon chains (three and four carbons long) are found in valine, leucine and isoleucine. These larger aliphatic side chains are hydrophobic.

Preferably the amino acid (i) is selected from valine, leucine and isoleucine, and mixtures thereof. Isoleucine is the most preferred amino acid (i) for the use according to the invention.

Simple derivatives of the amino acid (i) may be employed, such as salts and hydrosalts.

The total amount of amino acid (i) may suitably range from 0.001% to 5% by weight based on total weight of the hair treatment composition in which it is employed. Preferably, the amount of amino acid (i) is at least 0.01%, and will ideally range from 0.01% to 2% by weight based on total weight.

### Amino acid (ii)

Amino acids (ii) having an basic side chain may be selected from lysine, arginine and histidine and mixtures thereof. These amino acids are hydrophilic due to their polar side chains. Lysine and arginine are positively charged at neutral pH, whereas histidine can be uncharged or positively charged depending on its local environment.

Preferably the amino acid (ii) is selected from lysine and arginine, and mixtures thereof. Lysine is the most preferred amino acid (ii) for the use according to the invention.

Simple derivatives of the amino acid (ii) may be employed, such as salts and hydrosalts.

The total amount of amino acid (ii) may suitably range from 0.01% to 10% by weight based on total weight of the hair treatment composition in which it is employed. Preferably, the amount of amino acid (i) is at least 0.1%, and will ideally range from 0.1% to 5% by weight based on total weight.

We have surprisingly found that the combination of amino acids (i) and (ii) has a beneficial synergistic interaction with respect to the repair and/or prevention of split ends in hair, which significantly exceeds the efficacy of either class of amino acid when used in isolation at the same levels or even at higher levels.

### Product Form

Advantageously, compositions comprising amino acids (i) and (ii) for use according to the invention can be formulated as a shampoo and will then accordingly comprise one or more cleansing surfactants which are cosmetically acceptable and suitable for topical application to the hair.

Suitable cleansing surfactants, which may be used singularly or in combination, are selected from anionic, amphoteric and zwitterionic surfactants, and mixtures thereof.

Examples of anionic surfactants are the alkyl sulphates, alkyl ether sulphates, alkaryl sulphonates, alkanoyl isethionates, alkyl succinates, alkyl sulphosuccinates, N-alkyl sarcosinates, alkyl phosphates, alkyl ether phosphates, alkyl ether carboxylates, and alpha-olefin sulphonates, especially their sodium, magnesium, ammonium and mono-, di- and triethanolamine salts. The alkyl and acyl groups generally contain from 8 to 18 carbon atoms and may be unsaturated. The alkyl ether sulphates, alkyl ether phosphates and alkyl ether carboxylates may contain from 1 to 10 ethylene oxide or propylene oxide units per molecule.

Preferred anionic surfactants include sodium oleyl succinate, ammonium lauryl sulphosuccinate, ammonium lauryl sulphate, sodium dodecylbenzene sulphonate, triethanolamine dodecylbenzene sulphonate, sodium cocoyl isethionate, sodium lauryl isethionate and sodium N-lauryl sarcosinate. The most preferred anionic surfactants are sodium lauryl sulphate, triethanolamine monolauryl phosphate, sodium lauryl ether sulphate 1 EO, 2EO and 3EO, ammonium lauryl sulphate and ammonium lauryl ether sulphate 1EO, 2EO and 3EO.

Examples of amphoteric and zwitterionic surfactants include alkyl amine oxides, alkyl betaines, alkyl amidopropyl betaines, alkyl sulphobetaines (sultaines), alkyl glycinates, alkyl carboxyglycinates, alkyl amphopropionates, alkylamphoglycinates, alkyl amidopropyl hydroxysultaines, acyl taurates and acyl glutamates, wherein the alkyl and acyl groups have from 8 to 19 carbon atoms. Preferred amphoteric and zwitterionic surfactants include lauryl amine oxide, cocodimethyl sulphopropyl betaine and preferably lauryl betaine, cocamidopropyl betaine and sodium cocamphopropionate.

The cleansing surfactant(s) may be present in shampoo compositions for use according to the invention in a total amount of from about 1 to about 40% by weight based on the total weight of the shampoo composition, preferably from about 2 to about 30% by weight, optimally from about 10% to 30% by weight.

Shampoos for use according to the invention can also include nonionic surfactants to help impart aesthetic, physical or cleansing properties to the composition. The nonionic surfactant can be included in an amount ranging from 0% to about 5% by weight based on total weight.

For example, representative nonionic surfactants include condensation products of aliphatic ($C_8$ - $C_{18}$) primary or secondary linear or branched chain alcohols or phenols with alkylene oxides, usually ethylene oxide and generally having from 6 to 30 ethylene oxide groups.

Other representative nonionics include mono- or di-alkyl alkanolamides. Examples include coco mono- or diethanolamide and coco mono-isopropanolamide.

Further nonionic surfactants which can be included are the alkylpolyglycosides (APGs). Typically, the APG is one which comprises an alkyl group connected (optionally via a bridging group) to a block of one or more glycosyl groups. Preferred APGs are defined by the following formula:

$$RO - (G)_n$$

wherein R is a branched or straight chain alkyl group which may be saturated or unsaturated and G is a saccharide group.

R may suitably represent a mean alkyl chain length of from about $C_5$ to about $C_{20}$. Preferably R represents a mean alkyl chain length of from about $C_8$ to about $C_{12}$. Most preferably the value of R lies between about 9.5 and about 10.5. G may be selected from $C_5$ or $C_6$ monosaccharide residues, and is preferably a glucoside. G may be selected from the group comprising glucose, xylose, lactose, fructose, mannose and derivatives thereof. Preferably G is glucose.

The degree of polymerisation, n, may have a value of from about 1 to about 10 or more. Preferably, the value of n lies in the range of from about 1.1 to about 2. Most preferably the value of n lies in the range of from about 1.3 to about 1.5.

Suitable alkyl polyglycosides are commercially available and include for example those materials identified as: Oramix NS10 ex Seppic; Plantaren 1200 and Plantaren 2000 ex Henkel.

Likewise shampoos for use according to the invention can include other emulsifiers, conditioning agents, inorganic salts, humectants and similar materials to provide the composition with desirable aesthetic or physical properties.

Representative conditioning agents include silicones. Silicones are particularly preferred conditioning agents for hair. Representative silicones include volatile and nonvolatile silicones, such as for example polyalkylsiloxanes (optionally end-capped with one or more hydroxyl groups), polyalkylaryl siloxanes, siloxane gums and resins, cyclomethicones, aminofunctional silicones, quaternary silicones and mixtures thereof.

Preferred silicones include polydimethylsiloxanes (of CTFA designation dimethicone), siloxane gums, aminofunctional silicones (of CTFA designation amodimethicone) and hydroxylated polydimethylsiloxanes (of CTFA designation dimethiconol).

Various methods of making emulsions of particles of silicones are available and are well known and documented in the art.

Suitable silicone emulsions are commercially available in a pre-emulsified form. This is particularly preferred since the pre-formed emulsion can be incorporated into the shampoo composition by simple mixing.

Examples of suitable pre-formed emulsions include emulsions DC2-1310, DC2-1865, DC2-1870, DC2-1766 and DC2-1784, available from Dow Corning. These are emulsions of dimethiconol. Siloxane gums are also available in a pre-emulsified form, which is advantageous for ease of formulation. A preferred example is the material available from Dow Corning as DC X2-1787, which is an emulsion of cross-linked dimethiconol gum.

The amount of silicone incorporated into compositions for use according to the invention depends on the level of conditioning desired and the material used. A preferred amount is from 0.01 to about 10% by weight of the total composition although these limits are not absolute. The lower limit is determined by the minimum level to achieve conditioning and the upper limit by the maximum level to avoid making the hair and/or skin unacceptably greasy. We have found that an amount of silicone of from 0.5 to 1.5% by weight of the total composition, is a particularly suitable level.

A further preferred class of conditioning agents are per-alk(en)yl hydrocarbon materials, used to enhance the body, volume and stylability of hair.

EP 567 326 and EP 498 119 describe suitable peralk(en)yl hydrocarbon materials for imparting stylability and enhanced body to hair. Preferred materials are polyisobutylene materials available from Presperse, Inc. under the PERMETHYL trade name.

The amount of per-alk(en)yl hydrocarbon material incorporated into compositions for use according to the invention depends on the level of body and volume enhancement desired and the specific material used. A preferred amount is from 0.01 to about 10% by weight of the total composition although these limits are not absolute. The lower limit is determined by the minimum level to achieve the body and volume enhancing effect and the upper limit by the maximum level to avoid making the hair unacceptably stiff. We have found that an amount of per-alk(en)yl hydrocarbon material of from 0.5 to 2% by weight of the total composition is a particularly suitable level.

Shampoo compositions for use according to the invention may also include a polymeric cationic conditioning compound that is substantive to the hair and imparts conditioning properties to the hair.

The polymeric cationic conditioning compound will generally be present at levels of from 0.01 to 5%, preferably from about 0.05 to 1%, more preferably from about 0.08% to about 0.5% by weight. Synthetic or naturally derived polymers having a quaternised nitrogen atom are useful. The molecular weight of the polymer will generally be between 5 000 and 10 000 000, typically at least 10 000 and preferably in the range 100 000 to about 2 000 000.

Representative synthetic quaternised polymers include, for example: cationic copolymers of 1-vinyl-2-pyrrolidine and 1-vinyl-3-methyl-imidazolium salt (e.g., Chloride salt) (referred to in the industry by the Cosmetic, Toiletry, and Fragrance Association, "CTFA". as Polyquaternium-16); copolymers of 1-vinyl-2-pyrrolidine and dimethylaminoethyl methacrylate (referred to in the industry by CTFA as Polyquaternium-11); cationic diallyl quaternary ammonium-containing polymers including, for example, dimethyldiallylammonium chloride homopolymer (referred to in the industry (CTFA) as Polyquaternium 6); mineral acid salts of amino-alkyl esters of homo-and co-polymers of unsaturated carboxylic acids having from 3 to 5 carbon atoms, as described in U.S. Patent 4,009,256; and cationic polyacrylamides as described in WO95/22311.

Representative naturally-derived quaternised polymers include quaternised cellulosic compounds and cationic guar gum derivatives, such as guar hydroxypropyltrimonium chloride. Examples are JAGUAR C-13S, JAGUAR C-15, and JAGUAR-C17, commercially available from Meyhall in their JAGUAR trademark series.

Compositions for use in accordance with the invention may also be formulated as a hair conditioner for the treatment of hair (typically after shampooing) and subsequent rinsing. Such formulations will then accordingly comprise one or more conditioning surfactants which are cosmetically acceptable and suitable for topical application to the hair.

Suitable conditioning surfactants are selected from cationic surfactants, used singly or in admixture. Examples include quaternary ammonium hydroxides or salts thereof, e.g chlorides.

Suitable cationic surfactants include cetyltrimethylammonium chloride, behenyltrimethylammonium chloride, cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, cocotrimethylammonium chloride, and the corresponding hydroxides thereof. Further suitable cationic surfactants include those materials having the CTFA designations Quaternium-5, Quaternium-31 and Quaternium-18. Mixtures of any of the foregoing materials may also be suitable. A particularly useful cationic surfactant for use in hair conditioners of the invention is cetyltrimethylammonium chloride, available commercially, for example as GENAMIN CTAC, ex Hoechst Celanese.

In hair conditioners for use according to the invention, the level of cationic surfactant is preferably from 0.01 to 10%, more preferably 0.05 to 5%, most preferably 0.1 to 2% by weight of the composition.

Conditioners for use according to the invention advantageously incorporate a fatty alcohol material. The combined use of fatty alcohol materials and cationic surfactants in conditioning compositions is believed to be especially advantageous, because this leads to the formation of a lamellar phase, in which the cationic surfactant is dispersed.

Representative fatty alcohols comprise from 8 to 22 carbon atoms, more preferably 16 to 20. Examples of suitable fatty alcohols include cetyl alcohol, stearyl alcohol and mixtures thereof.

The level of fatty alcohol materials is conveniently from 0.01 to 10%, preferably from 0.1 to 5% by weight of the composition. The weight ratio of cationic surfactant to fatty alcohol is suitably from 10:1 to 1:10, preferably from 4:1 to 1:8, optimally from 1:1 to 1:4.

Conditioners for use according to the invention can include other emulsifiers, conditioning agents, inorganic salts, humectants and similar materials to provide the composition with desirable aesthetic or physical properties. Silicones, as described above for shampoo compositions, are particularly preferred conditioning agents for hair.

As further optional components for inclusion in shampoo or conditioner compositions for use according to the invention, in addition to water, may be mentioned the following conventional adjunct materials known for use in cosmetic compositions: suspending agents, thickeners, pearlescing agents, opacifiers, salts, perfumes, buffering agents, colouring agents, emollients, moisturisers, foam stabilisers, sunscreen materials, antimicrobial agents, preservatives, antioxidants, natural oils and extracts, propellants.

The invention will now be further illustrated by the following, non-limiting Examples.

<u>EXAMPLES</u>

<u>Examples 1 & 2</u>

The following formulations illustrate shampoo compositions according to the invention.

| INGREDIENT | % (by weight based on total weight) | |
|---|---|---|
| | Ex.1 | Ex.2 |
| Sodium lauryl ether sulphate (2EO); 70%active | 20 | 20 |
| Cocamidopropylbetaine | 6.67 | 6.4 |
| Sodium benzoate | 0.5 | 0.5 |
| Perfume | 0.55 | 0.55 |
| Vitamim E acetate | 0.05 | 0.05 |
| Titanium dioxide coated mica (TIMIRON MP 1001 ex Merck) | 0.2 | 0.2 |
| Silicone emulsion (DC 1784 ex Dow Corning) | 0.8 | 0.4 |
| Guar hydroxypropyltrimonium chloride (JAGUAR C13S ex Meyhall) | 0.1 | 0.1 |
| Citric acid | 0.42 | 0.42 |
| Sodium chloride | 0.7 | 0.7 |
| Sodium hydroxide | 0.15 | 0.15 |
| Isoleucine | 0.01 | 0.01 |
| Lysine | 0.1 | 0.1 |
| CARBOPOL 980 (Crosslinked polyacrylate ex BF Goodrich) | 0.4 | 0.4 |
| Water | to 100% | to 100% |

The shampoos of Examples 1 and 2 are both opacified liquids of viscosity 4000 to 6000 cps and pH 4.5 to 5. Both shampoos imparted excellent split ends reduction to treated hair.

Example 3 & 4

The following formulations illustrate post-wash hair conditioning compositions according to the invention.

| INGREDIENT | % (by weight based on total weight) | |
|---|---|---|
| | Ex.3 | Ex.4 |
| Cetyl trimethylammonium chloride | 2.4 | 2.4 |
| Cetearyl alcohol | 3.0 | 2.3 |
| Paraffin 52/54 | 1.00 | - |
| Glyceryl stearate | 0.7 | - |
| Silicone emulsion (DC 1784 ex Dow Corning) | 3.0 | 3.0 |
| Hydrophobically modified Hydroxyethylcellulose (POLYSURF 67 ex Aqualon) | - | 0.03 |
| Perfume | 0.5 | 0.5 |
| Vitamin E acetate | 0.05 | 0.05 |
| Preservative | 0.2 | 0.2 |
| Phenoxyethanol | 0.4 | - |
| Isoleucine | 0.01 | 0.01 |
| Lysine | 0.1 | 0.1 |
| Citric acid | - | 0.025 |
| Water | to 100% | to 100% |

The conditioner of Example 2 has a pH of 4.5-5.0 and that of Example 3 has a pH of 3.0 to 3.5. Both are opacified liquids of viscosity 7000-11000 cps.

Both conditioners imparted excellent split ends reduction to treated hair.

**Claims**

1.  The use in a topical hair treatment composition of a combination of

    (i) an amino acid having an aliphatic side chain; and
    (ii) an amino acid having a basic side chain

    for the repair and/or prevention of split ends in hair.

2.  The use according to claim 1, in which (i) is isoleucine.

3.  The use according to claim 1 or 2, in which the amount of amino acid (i) is at least 0.01% by weight based on total weight.

4.  The use according to any of claims 1 to 3, in which (ii) is lysine.

5.  The use according to any of claims 1 to 4, in which the amount of amino acid (ii) is at least 0.1% by weight based on total weight.

6.  The use according to any of claims 1 to 5, in which the topical hair treatment composition is formulated as a shampoo composition.

7.  The use according to any of claims 1 to 5, in which the topical hair treatment composition is formulated as a hair conditioner.

**Patentansprüche**

1.  Verwendung einer Kombination aus

    (i) einer Aminosäure mit einer aliphatischen Seitenkette; und
    (ii) einer Aminosäure mit einer basischen Seitenkette

    in einer topischen Haarbehandlungszusammensetzung zur Reparatur und/oder Vorbeugung gespaltener Spitzen im Haar.

2.  Verwendung nach Anspruch 1, worin (i) Isoleucin ist.

3.  Verwendung nach Anspruch 1 oder 2, worin die Menge an Aminosäure (i) mindestens 0,01 Gew.-%, basierend auf dem Gesamtgewicht, beträgt.

4.  Verwendung nach einem der Ansprüche 1 bis 3, worin (ii) Lysin ist.

5.  Verwendung nach einem der Ansprüche 1 bis 4, worin die Menge an Aminosäure (ii) mindestens 0,1 Gew.-%, basierend auf dem Gesamtgewicht, beträgt.

6.  Verwendung nach einem der Ansprüche 1 bis 5, worin die topische Haarbehandlungszusammensetzung als eine Shampoozusammensetzung formuliert wird.

7.  Verwendung nach einem der Ansprüche 1 bis 5, worin die topische Haarbehandlungszusammensetzung als eine Harrspülung formuliert wird.

**Revendications**

1. Utilisation dans une composition de traitement capillaire topique d'une combinaison de

    (i) un acide aminé ayant une chaîne latérale aliphatique ; et
    (ii) un acide aminé ayant une chaîne latérale basique

    pour la réparation et/ou la prévention des extrémités fourchues des cheveux.

2. Utilisation selon la revendication 1, dans laquelle (i) est l'isoleucine.

3. Utilisation selon la revendication 1 ou 2, dans laquelle la quantité d'acide aminé (i) est d'au moins 0,01 % en poids sur la base du poids total.

4. Utilisation selon l'une quelconque des revendications 1 à 3, dans laquelle (ii) est la lysine.

5. Utilisation selon l'une quelconque des revendications 1 à 4, dans laquelle la quantité d'acide aminé (ii) est d'au moins 0,1 % en poids sur la base du poids total.

6. Utilisation selon l'une quelconque des revendications 1 à 5, dans laquelle la composition de traitement capillaire topique est formulée sous forme de composition de shampoing.

7. Utilisation selon l'une quelconque des revendications 1 à 5, dans laquelle la composition de traitement capillaire topique est formulée sous forme de conditionneur capillaire.